# EUROPÄISCHE PATENTSCHRIFT

(11) **EP 3 952 843 B1**
(45) Veröffentlichungstag und Bekanntmachung des Hinweises auf die Patenterteilung: **06.08.2025**
(21) Anmeldenummer: 20722226.6
(22) Anmeldetag: 09.04.2020
(51) Int. Cl.: A61K 9/16, A61K 31/00

(54) **VERFAHREN ZUR KONTINUIERLICHEN HERSTELLUNG EINES WIRKSTOFFGRANULATS**
METHOD FOR CONTINUOUS PRODUCTION OF A GRANULATE AGENT
PROCÉDÉ DE FABRICATION EN CONTINU D'UN GRANULAT DE SUBSTANCE ACTIVE

(30) Priorität: 11.04.2019 EP 19168673
(43) Veröffentlichungstag der Anmeldung: 16.02.2022
(73) Patentinhaber: ADD Advanced Drug Delivery Technologies, Ltd., 4133 Pratteln (CH)
(72) Erfinder: NOWAK, Reinhard, 79589 Binzen (DE); PÖLLINGER, Norbert, 79379 Müllheim (DE); GRAVE, Annette, 79541 Lörrach (DE); JACOB, Michael, 99427 Weimar (DE)
(74) Vertreter: Breuer, Markus
(86) Internationale Anmeldenummer: PCT/EP2020/060277
(87) Internationale Veröffentlichungsnummer: WO 2020/208201

(56) Entgegenhaltungen:
- WO-A1-2015/012365
- US-A- 6 159 252
- PALIS STEFAN ET AL: "Online parameter identification for continuous fluidized bed spray granulation with external sieve-mill cycle", 2017 22ND INTERNATIONAL CONFERENCE ON METHODS AND MODELS IN AUTOMATION AND ROBOTICS (MMAR), IEEE, 28 August 2017 (2017-08-28), pages 594 - 598, XP033154808, DOI: 10.1109/MMAR.2017.8046895

## Beschreibung

### Gebiet der Erfindung

Die Erfindung betrifft ein Verfahren zur kontinuierlichen Herstellung eines Wirkstoffgranulats, das Granulat selbst sowie dessen Verwendung. Die Erfindung betrifft auch Darreichungsformen mit kontrollierter Freisetzung. Als Wirkstoff kommen Metformin und seine Säureadditionssalze, insbesondere Metformin-Hydrochlorid, zum Einsatz.

### Hintergrund der Erfindung

Pharmazeutische Wirkstoffe fallen nach der Herstellung und Reinigung in der Regel in einer Form an, die umfangreiche weitere Aufarbeitungsschritte erfordert, um den Wirkstoff in eine Darreichungsform zu überführen. Insbesondere fällt der Wirkstoff oftmals nicht in Form von Teilchen an, die ohne weiteres formuliert werden können.

Zu den nötigen Aufarbeitungsschritten gehören Zerkleinern, Mahlen, Sieben und dergleichen. Es ist auch bekannt, Wirkstoffpulver durch Sprühtrocknung von Wirkstofflösungen herzustellen.

Wirkstoffpulver müssen aber in aller Regel auch weiterverarbeitet werden, bevor die Herstellung von Darreichungsformen, wie Tabletten, möglich ist, denn feinteilige Formen von Wirkstoffen sind mit verarbeitungstechnischen Nachteilen behaftet. Zu diesen Nachteilen gehört eine oftmals mangelnde Fließfähigkeit. Ein weiterer Nachteil besteht darin, dass feine Pulver häufig eine geringe Stabilität aufweisen. Sie neigen zu Aggregation und Verklumpung.

Um solche Nachteile zu vermeiden, ist es üblich, Granulate bereitzustellen.

Granulate, die einen Wirkstoff und ggf. einen oder mehrere Hilfsstoffe enthalten, können zur Herstellung von Darreichungsformen verwendet werden, indem sie beispielsweise allein oder zusammen mit weiteren Komponenten zu Tabletten verpresst werden. Wirkstoffhaltige Teilchen können darüber hinaus eingefüllt in Kapseln oder in Form eines Pulvers für eine Suspension oder Lösung verwendet werden. Sie können auch mit Überzügen versehen werden.

Zur Herstellung von Granulaten sind eine Reihe von Verfahren bekannt. Diese Verfahren arbeiten häufig als Batchverfahren. Zum Einsatz kommt ein vorverarbeiteter, in der Regel gemahlener und gesiebter Wirkstoff.

Die Eigenschaften der erhaltenen Granulate sind nicht immer zufriedenstellend, insbesondere im Hinblick auf Fließfähigkeit und Stabilität.

Der Stand der Technik enthält weiterhin Vorschläge, Granulate unter Verwendung von Strahlschichtapparaten zu erzeugen. So ist aus DE 103 22 062 A1 bekannt, Granulate verschiedenartiger Materialien durch Einbringen von Flüssigkeiten in eine Feststoffströmung eines Strahlschichtapparates herzustellen. Die genannte Anmeldung beschäftigt sich jedoch weder mit den Besonderheiten pharmazeutischer Wirksubstanzen noch mit den Bedingungen, die geeignet sind, um solche Substanzen zu verarbeiten.

DE 100 04 939 C1 betrifft eine steuerbare Gasanströmeinrichtung für Strahlschichtapparate.

WO 2004/108911 A2 beschreibt Herstellungsverfahren für Enzymgranulate sowie derartige Granulate. Zu der Herstellung wird ein Strahlschichtapparat eingesetzt. Mit der Herstellung von Tabletten und der Tablettierbarkeit der Granulate beschäftigt sich die Anmeldung nicht.

WO 2008/110374 A2 betrifft Pellets enthaltend eine pharmazeutische Substanz mit einer Bruchfestigkeit von mehr als 0,001 Newton, Verfahren zu deren Herstellung sowie pharmazeutische Präparate auf Basis solcher Pellets. Es wird gezeigt, dass kugelförmige Mannitolpellets mit einheitlicher Kornverteilung und glatter Oberfläche ausgehend von einer Mannitollösung hergestellt werden können und dass solche Pellets durch Wirkstofflayering mit einer Wirkstoffschicht überzogen werden können.

Während die vorstehenden Dokumente keinen Hinweis auf Metformin enthalten, ist die Herstellung von Darreichungsformen, die Metformin oder dessen Säureadditionssalze, insbesondere Metformin-Hydrochlorid, enthalten, Gegenstand zahlreicher Patentanmeldungen und Veröffentlichungen.

O.R. Arndt und P. Kleinebudde, AAPS PharmSciTech. 2018 Jul;19(5):2068-2076, weisen darauf hin, dass Metformin eine schlechte Tablettierbarkeit und eine schlechte Fließfähigkeit aufweist und daher typischerweise vor der Tablettierung mit einem Bindemittel nassgranuliert wird. Dies wird wegen der damit verbundenen Kosten aber als nachteilig angesehen. Es wird daher ein Trockenverfahren mittels Walzenverdichtung vorgeschlagen.

US 6 667 054 B2 beschreibt Tabletten, die Metformin-Hydrochlorid enthalten. Sie werden aus einer trockenen Mischung aus Metformin-Hydrochlorid und Methylcellulose hergestellt.

US 6 117 451 beschreibt ein Gemisch aus einem kristallinen Metformin-Hydrochlorid-Pulver und pulverförmigen Hilfsstoffen, das direkt zu Tabletten verpresst werden kann.

H. Takasaki et al., Results in Pharma Sciences 5 (2015) 1-7 beschreiben eine feuchtigkeitsaktivierte Trockengranulation von Metformin-Hydrochlorid.

B.S. Barot et al., Acta Pharm. 60 (2010) 165-175, weisen darauf hin, dass Metformin-Hydrochlorid hygroskopisch ist und Stabilitätsprobleme aufweist, und beschreiben die Entwicklung eines direkt verpressbaren Metformin-Hydrochlorids durch Sprühtrocknen. Dies führt zu einem Produkt mit annähernd kugelförmigen Teilchen, die typischerweise einen Durchmesser von weniger als 50 µm aufweisen.

Des Weiteren sind Untersuchungen zur Freisetzung von Metformin aus Darreichungsformen durchgeführt worden, um festzustellen, wo im Gastrointestinaltrakt der Wirkstoff freigesetzt werden sollte, um eine gute Wirkung zu erzielen. Dabei ist insbesondere vorgeschlagen worden, dass die Freisetzung erst in tieferen Darmabschnitten besonders vorteilhaft ist (H. Schatz, Neue Erkenntnisse zu Metformin (2016). https://www.diabsite.de/aktuelles/nachrichten/2016/160503b.html).

Die Veröffentlichung von Palis und Kienle mit dem Titel "Online parameter identification for continuous fluidized bed spray granulation with external sieve-mill cycle" (22nd International Conference on Methods and Models in Automation and Robotics (MMAR), IEEE, 28. August 2017 Seiten 594-598) beschäftigt sich mit numerischen Simulationen im Zusammenhang mit einer kontinuierlichen Wirbelschicht-Sprühgranulation mit einem externen Sieb- und Mahlkreislauf.

US 6 159 252 A betrifft ein Verfahren zur Herstellung bestimmter Granulate durch Wirbelschicht-Sprühgranulation. Beispielhaft wird die Herstellung von Natriumpercarbonat beschrieben.

WO 2015/012365 A1 betrifft ein pharmazeutisches Präparat, das einen DPP-IV-Inhibitor und Metformin umfasst und in dem der darin enthaltene DPPIV-Inhibitor chemisch stabilisiert ist.

Ungeachtet all dieser Vorschläge besteht aber weiterhin ein Bedarf an verbesserten Verfahren zur Herstellung von Metformin enthaltenden Darreichungsformen.

### Aufgaben und Kurzdarstellung der Erfindung

Eine Aufgabe der Erfindung besteht darin, ein kontinuierliches Verfahren zur Herstellung eines Wirkstoffgranulats bereitzustellen, wobei es sich bei dem Wirkstoff um Metformin oder ein Säureadditionssalz davon, insbesondere Metformin-Hydrochlorid, handelt. Das Verfahren soll einen hohen Durchsatz und eine hohe Ausbeute bei einstellbaren Granulateigenschaften (wie Partikeldurchmesser, Feuchte, Schüttdichte) ermöglichen.

Eine weitere Aufgabe besteht darin, ein Verfahren bereitzustellen, das es ermöglicht, die Teilchengröße der Granulatteilchen einzustellen.

Darüber hinaus besteht eine Aufgabe der Erfindung darin, mindestens einen Wirkstoff enthaltende Granulatteilchen herzustellen, die eine gute Fließfähigkeit zeigen.

Zudem besteht eine Aufgabe darin, ein Granulat mit einer hohen Stabilität bereitzustellen. Insbesondere sollen die Granulatteilchen nicht aggregieren oder verklumpen.

Eine weitere Aufgabe besteht darin, ein Verfahren zur Herstellung eines Halbfertigprodukts anzugeben, wobei das Halbfertigprodukt aus einem Wirkstoff und mindestens einem Hilfsstoff besteht und vorzugsweise zu Tabletten weiterverarbeitet werden kann.

Eine Aufgabe besteht auch darin, ein Verfahren zur Herstellung von Tabletten bereitzustellen.

Schließlich besteht eine Aufgabe darin, Darreichungsformen mit kontrollierter Freisetzung und Verfahren zu deren Herstellung bereitzustellen, insbesondere Darreichungsformen, die den Wirkstoff erst in tiefen Darmabschnitten, wie dem Ileum oder dem Colon, freisetzen.

Erfindungsgemäß wurde nun festgestellt, dass die kontinuierliche Herstellung eines wirkstoffhaltigen Granulats möglich ist, indem Tröpfchen einer Lösung oder Suspension, die den Wirkstoff enthält, in einen Prozessraum eingebracht werden, in dem Flüssigkeit verdampft, wobei die Tröpfchen mit Hilfe eines geeignet temperierten Prozessgases so geführt werden, dass Teilchen, die sich bereits im Prozessraum befinden, mit Tröpfchen, die zumindest noch so viel Flüssigkeit enthalten, dass sie an die Teilchen angelagert werden, in Kontakt kommen.

Das erfindungsgemäße Verfahren zur kontinuierlichen Herstellung eines Wirkstoffgranulats umfasst demnach folgende Stufen:
(a) Herstellen einer Sprühzusammensetzung, indem ein Wirkstoff und ggf. ein oder mehrere Hilfsstoffe in einer Flüssigkeit gelöst oder dispergiert werden;
(b) Bereitstellen von festen Teilchen in einem Prozessraum;
(c) Einführen von Tröpfchen aus der Sprühzusammensetzung in eine Eindüsungszone des Prozessraums, in dem die Flüssigkeit verdampft;
(d) wiederholtes Vorbeiführen der festen Teilchen an eingesprühten Tröpfchen in dem Prozessraum mit Hilfe eines Prozessgasstrahls, so dass zumindest ein Anteil der Tröpfchen, die bereits einen Teil der enthaltenen Flüssigkeit verloren haben können, mit festen Teilchen in Kontakt kommt und durch Anlagerung größere feste Teilchen gebildet werden;
(e) Entnehmen des Wirkstoffgranulats aus dem Prozessraum in Form von festen Teilchen,
wobei der Wirkstoff Metformin oder ein Säureadditionssalz von Metformin, insbesondere Metformin-Hydrochlorid, umfasst.

Im Unterschied zur Herstellung von Teilchen durch Sprühtrocknen in einem herkömmlichen Sprühturm werden die sich bildenden Teilchen erfindungsgemäß in dem Prozessraum solange im Kreis geführt, bis sie durch wiederholte Anlagerung von Tröpfchen der Lösung oder Dispersion und Verdampfen der Flüssigkeit die gewünschte Größe erreicht haben.

Bei dem erfindungsgemäßen Verfahren kann also das Wachstum der Teilchen gesteuert werden.

Das erhaltene Granulat kann zu Tabletten verarbeitet werden. Es hat im Vergleich mit bekannten Granulaten verbesserte Eigenschaften, insbesondere im Hinblick auf Stabilität und Fließfähigkeit.

Das Granulat kann auch zu überzogenen Darreichungsformen verarbeitet werden, insbesondere Darreichungsformen, die den Wirkstoff erst im Ileum oder im Colon freisetzen.

### Kurze Beschreibung der Figuren

Die Erfindung wird nachstehend unter Bezugnahme auf Figuren näher erläutert.

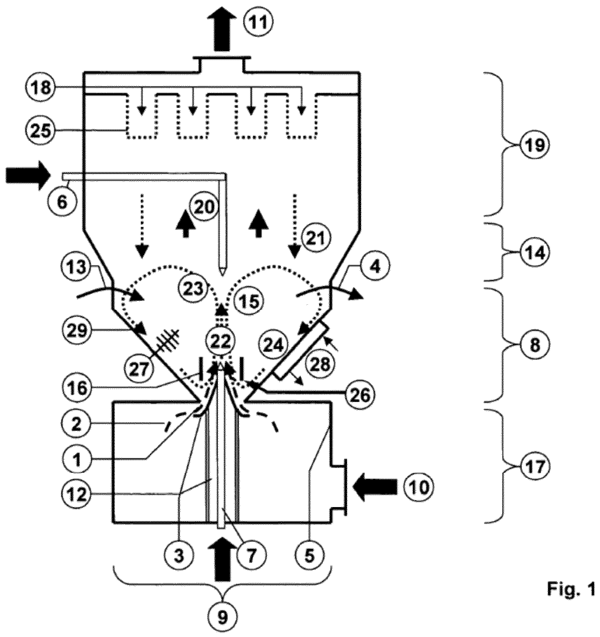

In Fig. 1 ist schematisch eine Anlage zur Durchführung des erfindungsgemäßen Verfahrens dargestellt.
Fig. 2 zeigt eine typische Teilchengrößenverteilung.
Fig. 3 zeigt eine mikroskopische Aufnahme einer typischen Probe.

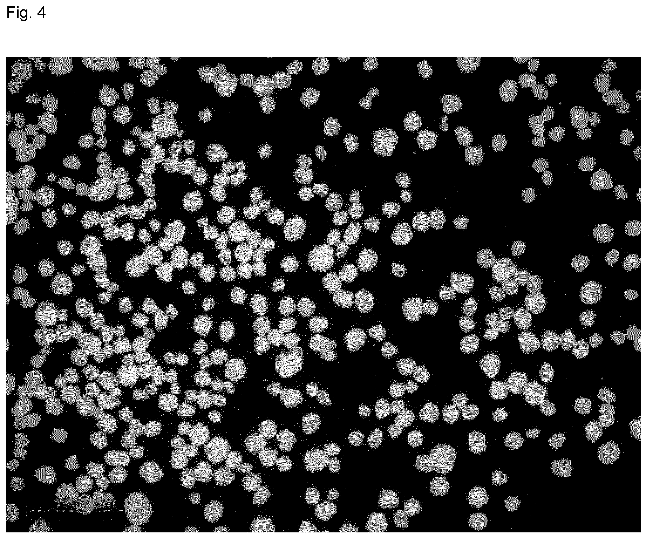

Fig. 4 zeigt eine mikroskopische Aufnahme einer weiteren Probe.

### Ausführliche Darstellung der Erfindung

Die erfindungsgemäß hergestellten Granulatteilchen enthalten einen Wirkstoff. Der Wirkstoff umfasst Metformin oder eines seiner Säureadditionssalze, insbesondere Metformin-Hydrochlorid. Gemäß einer Ausführungsform handelt es sich bei dem Wirkstoff um Metformin oder eines seiner Säureadditionssalze, insbesondere Metformin-Hydrochlorid.

Gemäß einer Ausführungsform bestehen die Granulatteilchen aus dem Wirkstoff.

Die Granulatteilchen können aber zusätzlich zu dem Wirkstoff auch einen oder mehrere Hilfsstoffe enthalten. Als Hilfsstoff kann jeder pharmazeutisch geeignete Hilfsstoff eingesetzt werden. Insbesondere kommen Hilfsstoffe zum Einsatz, die typischerweise für die Granulat- und Tablettenherstellung verwendet werden. Beispielhafte Hilfsstoffe sind Bindemittel, Gleitmittel, Sprengmittel und Füllstoffe.

Ein bevorzugter Hilfsstoff ist ein Bindemittel. Bindemittel fördern die Bindung der Granulatteilchen bei der Tablettierung.

In einer Ausführungsform unterstützen sie auch die Bildung der Granulatteilchen, insbesondere wenn Wirkstoff in der Flüssigkeit ganz oder teilweise dispergiert ist.

Beispielhafte Bindemittel sind Polyvinylpyrrolidon (PVP), Vinylpyrrolidon-VinylacetatCopolymere, Hydroxypropylcellulose (HPC) und Hydroxypropylmethylcellulose (HPMC). Bevorzugt ist PVP.

Das Bindemittel kann beispielsweise in einer Menge von 0,1 bis 10 Gew.-%, vorzugsweise 1 bis 7 Gew.-% und insbesondere 2,5 bis 5 Gew.-%, bezogen auf den Trockenstoffgehalt, eingesetzt werden.

Bei dem erfindungsgemäßen Verfahren wird eine Sprühzusammensetzung in einen Prozessraum eingesprüht. Bei der Sprühzusammensetzung handelt es sich um eine Lösung oder eine Suspension.

Als Flüssigkeit zur Herstellung der Lösung oder Suspension kann jede Flüssigkeit verwendet werden, die mit dem Wirkstoff nicht oder nicht in wesentlichem Maße reagiert und die unter Bedingungen entfernt werden kann, die nicht oder nicht in wesentlichem Maße zur Zersetzung des Wirkstoffs führen.

Eine bevorzugte Flüssigkeit enthält Wasser. Insbesondere handelt es sich bei der Flüssigkeit um Wasser.

Die Sprühzusammensetzung enthält den Wirkstoff und ggf. einen oder mehrere Hilfsstoffe. Vorzugsweise enthält die Sprühzusammensetzung eine hohe Konzentration an Wirkstoff und/oder Hilfsstoffen.

Die Sprühzusammensetzung kann auch Bestandteile in ungelöster Form enthalten. In einer bevorzugten Ausführungsform ist die Sättigungslöslichkeit eines oder mehrerer der Bestandteile überschritten, so dass eine Suspension vorliegt.

Bei einem hohen Trockenstoffgehalt in der Sprühzusammensetzung muss weniger Flüssigkeit verdampft werden, um die gewünschten festen Teilchen zu erhalten, so dass ein höherer Durchsatz möglich wird. Ein hoher Trockenstoffgehalt ist deshalb bevorzugt. Ebenso ist es bevorzugt, eine Suspension zu verwenden.

Der Trockenstoffgehalt der Sprühzusammensetzung beträgt typischerweise mindestens 25 Gew.-%, vorzugsweise mindestens 40 Gew.-%, insbesondere mindestens 50 Gew.-% und ganz besonders bevorzugt mindestens 65 Gew.-%.

Der Trockenstoffgehalt bezieht sich dabei jeweils auf das Gesamtgewicht der eingesetzten Feststoffe, relativ zum Gesamtgewicht der Sprühzusammensetzung.

Der Anteil des Wirkstoffs an dem Trockenstoffgehalt beträgt typischerweise mindestens 70 Gew.-%, vorzugsweise mindestens 80 Gew.-% und insbesondere mindestens 90 Gew.-%. Er kann 100 Gew.-% betragen.

Bei dem erfindungsgemäßen Verfahren werden aus der Sprühzusammensetzung Tröpfchen gebildet. Die Tröpfchen aus der Lösung oder Suspension sind fließfähig.

Im Prozessraum verlieren sie durch Verdampfen an Flüssigkeit. Dabei können aus den Tröpfchen kleine feste Teilchen gebildet werden.

Für das erfindungsgemäße Verfahren ist aber kennzeichnend, dass Teilchen, die sich bereits im Prozessraum befinden, mit Tröpfchen, die zumindest noch so viel Flüssigkeit enthalten, dass sie an die Teilchen angelagert werden, in Kontakt kommen. Die Tröpfchen müssen bei Kontakt mit den festen Teilchen also zumindest an der Oberfläche verkleben.

Durch eine solche Anlagerung können Teilchen ausreichender Größe gebildet werden.

Dazu ist es wesentlich, dass der Aufbau der Teilchen ermöglicht wird, indem vorgelegte, also bereits in fester Form in den Prozessraum eingebrachte Teilchen, oder durch Einsprühen aus der Sprühzusammensetzung gebildete Teilchen wiederholt mit Tröpfchen der Sprühzusammensetzung in Kontakt kommen, so dass sich Aggregate bilden. Die erfindungsgemäß erzeugten festen Teilchen stellen typischerweise Aggregate aus fest miteinander verbundenen Kügelchen dar.

Bei dem erfindungsgemäßen Verfahren werden die Teilchen innerhalb des Prozessraumes mit Hilfe des definiert geführten Prozessgasstrahls bewegt, so dass eine zirkulierende Feststoffströmung erzeugt wird. Die Feststoffströmung führt in den Bereich der Vorrichtung (Eindüsungszone), in dem Tröpfchen eingebracht werden, die an feste Teilchen angelagert werden können.

Gemäß einer Ausführungsform können Teilchen, die eine gewünschte Größe erreicht haben, den Prozessraum verlassen. Kleinere Teilchen verbleiben im Prozessraum, so dass sie erneut mit Tröpfchen in Kontakt kommen können. Gemäß einer anderen Ausführungsform wird ein Anteil der festen Teilchen aus dem Prozessraum entnommen. Das entnommene Material wird klassiert, und kleine Teilchen können in den Prozessraum zurückgeführt werden. Ebenso können zu große Teilchen nach Zerkleinern in den Prozessraum zurückgeführt werden.

Bei dem Prozessgas kann es sich beispielsweise um Luft oder ein Inertgas, wie Stickstoff, Kohlendioxid oder ein Edelgas handeln.

Der Prozessgasstrahl ist sowohl für den Stofftransport als auch für den Wärmetransport wesentlich. Erfindungsgemäß wird die Temperatur des Prozessgasstrahls so gewählt, dass es zu einem Kontakt der eingesprühten Tröpfchen mit bereits verfestigten Teilchen unter Ausbildung größerer Teilchen kommt. Insbesondere wird für solche Temperaturbedingungen im Prozessraum gesorgt, dass das Produkt keinen die Stabilität beeinträchtigenden Temperaturbedingungen ausgesetzt wird, andererseits aber ein ausreichendes Trocknen durch Verdampfen von Flüssigkeit sichergestellt wird.

Typischerweise weist der Prozessgasstrahl eine Temperatur im Bereich von 60 bis 100°C auf. Die Produkttemperatur liegt typischerweise bei 30 bis 60°C.

Bevorzugt hat der Prozessgasstrahl eine Temperatur im Bereich von 70 bis 90°C. Die Produkttemperatur liegt bevorzugt bei 35 bis 50°C.

In einer besonders bevorzugten Ausführungsform liegt die Prozessgastemperatur bei 80°C und die Produkttemperatur bei 40°C.

Erfindungsgemäß werden Tröpfchen aus der Sprühzusammensetzung und feste Teilchen in einer Strahlschicht miteinander in Kontakt gebracht. Unter Strahlschicht wird verstanden, dass die vollständig fluidisierten festen Teilchen sich in einer zeitlich stabilen geschlossenen Feststoffströmung befinden. Die Strahlschicht wird mit Hilfe des definiert geführten Prozessgasstrahls erzeugt. Innerhalb der Strahlschicht sind drei Fluidisationszustände oder -zonen zu unterscheiden. In einer ersten Zone oder Auswurfzone erfolgt die Beschleunigung der festen Teilchen unter Einwirkung des definiert geführten Prozessgasstrahls, wobei sich die Teilchen in dieser Zone in der Strömungsrichtung des Prozessgasstrahls bewegen. Typischerweise wird der Prozessgasstrahl senkrecht nach oben geführt. Entsprechend herrscht in der Auswurfzone der Strahlschicht eine vorwiegend senkrecht nach oben gerichtete Strömung vor. In einer anschließenden zweiten Zone oder Fontänenzone ändern die Teilchen ihre Strömungsrichtung. Es herrscht vorwiegend eine Querströmung vor. Schließlich gelangen die Teilchen in eine dritte Zone oder Rücklaufzone. Dort weisen die Teilchen dann eine abwärtsgerichtete Bewegung auf, bis sie schließlich wieder in den Einfluss des definiert geführten Prozessgasstrahls gelangen und in der ersten Zone erneut von diesem mitgeführt werden. In der Rücklaufzone bewegen sich die Teilchen typischerweise unter dem Einfluss der Schwerkraft.

Das Versprühen der Sprühzusammensetzung kann durch Zwei- und Mehrstoffdüsen erfolgen. Weiterhin ist es möglich, das Versprühen durch Druckdüsen zu bewirken.

Alternativ kann ein Vertropfen durch Rotationszerstäuber, Strahlschneider, Ultraschallzertropfer und andere dem Fachmann bekannte Vorrichtungen erfolgen.

Erfindungsgemäß ist es möglich, durch Einsprühen von Tröpfchen einer Sprühzusammensetzung in den Prozessraum und Trocknen dieser Tröpfchen Keime aus festen Teilchen zu bilden, die dann mit weiteren Tröpfchen in Kontakt gebracht werden, um Teilchen der gewünschten Größe zu bilden. Alternativ oder zusätzlich können bei dem Verfahren feste Teilchen von außen zugeführt werden. Beispielsweise können aus dem Prozess entnommene zu kleine Teilchen in den Prozessraum als Keimmaterial zurückgeführt werden. Ebenso können zu große aus dem Prozess entnommene Teilchen oder Agglomerate von Teilchen durch ein beliebiges Zerkleinerungsaggregat zerkleinert und in den Prozessraum als Keimmaterial zurückgeführt werden.

Die nach dem erfindungsgemäßen Verfahren gebildeten Teilchen werden dem Prozessraum entnommen. Der Materialaustrag des Fertigproduktes aus dem Prozessraum oder ein Materialtransport in einen weiteren nachgeschalteten Prozessraum kann beispielsweise im Bereich des Übergangs der Querströmung zu der nach unten gerichteten Feststoffströmung erfolgen. Gemäß einer Ausführungsform werden die aus dem Prozessraum ausgetragenen Teilchen nicht klassiert. Gemäß einer anderen Ausführungsform werden die aus dem Prozessraum ausgetragenen Teilchen klassiert durch einen oder mehrere Sichtapparate entnommen.

Das erfindungsgemäße Verfahren kann beispielsweise mit Hilfe einer Vorrichtung durchgeführt werden, wie sie in DE 103 22 062 A1 beschrieben ist. Der Inhalt dieser Anmeldung wird durch Verweis zum Gegenstand der vorliegenden Anmeldung gemacht.

Vorzugsweise wird das erfindungsgemäße Verfahren unter Verwendung einer Vorrichtung, wie sie in der beigefügten Figur gezeigt ist, durchgeführt. Dies wird nachstehend ausführlich erläutert.

Das Prozessgas 10 (in der Regel erwärmte Luft) wird einer Zuluftkammer 17 mit rechteckigem Querschnitt 9 und begrenzenden Seitenwänden 5 zugeführt. In der Zuluftkammer 17 verteilt sich das Prozessgas 10 und tritt über Spaltöffnungen 1 in den Prozessraum 8 in Form von Gasstrahlen 2 ein. Der vorzugsweise horizontal in den Spalt 1 eintretende Prozessgasstrom wird durch das Umlenkteil 3 vorzugsweise nach oben in den Prozessraum 8 hinein umgelenkt und strömt als eine Art Freistrahl in den Apparat hinein. Im Weiteren kann sich der Apparatequerschnitt optional in der Expansionszone 14 vergrößern, so dass sich die Geschwindigkeit der Prozessgasströmung nach oben hin stetig verringert. Das Gas verlässt den Apparat als Abgas 11 oberhalb der Expansionszone 14 über das Abluftteil 19, in das optional ein Entstaubungssystem (z.B. Filterpatronen oder Textilfilterelemente) integriert werden kann.

Im Prozessraum 8 befindet sich eine Menge an Teilchen, die durch den Prozessgasstrahl nach oben hin mitgerissen werden. Zu Beginn des Verfahrens können feste Teilchen in den Prozessraum eingebracht werden; der Prozess kann aber auch dadurch gestartet werden, dass feste Teilchen aus eingesprühter Sprühzusammensetzung erzeugt werden.

Im oberen Bereich des Prozessraumes 8 sowie in der darüber befindlichen Expansionszone 14 nimmt die Gasgeschwindigkeit ab, so dass die aufwärts strömenden Teilchen seitlich aus dem Gasstrahl 23 heraustreten und in den Prozessraum 8 zurückfallen. Der Prozessraum 8 wird im unteren Bereich von geneigten Seitenwänden 29 begrenzt. Bedingt durch diese Seitenneigung werden die Teilchen unter Wirkung der Schwerkraft über die Rücklaufzone 24 in Richtung des Gaseintrittsspaltes 1 befördert, wo sie anschließend wieder vom Prozessgas in den Prozessraum 8 mitgerissen werden.

Durch diesen Mechanismus bildet sich eine sehr gleichförmige Feststoffzirkulation 15 bestehend aus einer Aufwärtsströmung und einem Rücklauf in Richtung des Prozessgaseintrittes. Dadurch liegt auch bei sehr geringen Mengen an Teilchen im Prozessraum 8 in der Kernzone oberhalb des Umlenkteiles 3 eine hohe Partikeldichte vor. In diesem Bereich werden ein oder mehrere Sprühdüsen 7 angeordnet, die gleichgerichtet zum Prozessgasstrahl nach oben sprühen und zum Einbringen der Sprühzusammensetzung dienen.

Durch die hohe Partikelbeladung in der Kernzone ergeben sich in der Bedüsungszone 22 sehr vorteilhafte Bedingungen für den Wärme- und Stoffübergang. Die Sprühzusammensetzung verliert durch Verdampfen rasch an Flüssigkeit. Durch Kontakt von festen Teilchen, die sich bereits im Prozessraum befinden, mit Tröpfchen der Sprühzusammensetzung, die bereits einen Teil der enthaltenen Flüssigkeit verloren haben können, kommt es zur Bildung von größeren Teilchen und Aggregaten von Teilchen.

Das Prozessgas kann einen Teil der Partikeln sowie Feingut und Stäube als feststoffbeladene Abluft 20 aus dem Prozessraum 8 ausgetragen. Zur Abscheidung dieser Teilchen können das im Abluftteil 19 optional integrierte Filtersystem oder dem Apparat nachgeschaltete Entstaubungsanlagen verwendet werden. Im Falle einer integrierten Entstaubungsanlage 25 können beispielsweise Druckluftimpulse 18 genutzt werden, um die zurückgehaltenen Partikeln als abgetrennten Feststoff 21 in den Prozessraum 8 zurückzuführen.

Im Vergleich zu Wirbelschichtapparaten mit integrierten Filteranlagen wird die Staubrückführung dadurch erleichtert, dass die aufwärts gerichtete Prozessgasströmung im Wesentlichen örtlich begrenzt ist und somit die zurückzuführenden Teilchen außerhalb des Gasstrahles sicher absinken können. Durch die Sogwirkung in der Nähe des Gaseintrittsspaltes 1 wird dieser Mechanismus zusätzlich gefördert. Alternativ können von der Abluft abgeschiedene Teilchen in den Prozessraum 8 zurückgeführt werden. Dazu können im unteren Bereich der geneigten Seitenwände 29 verschiedenartige Zuführungen 26 angeordnet sein. Bedingt durch die hohe Geschwindigkeit des Prozessgasstrahls in der Nähe des Gaseintrittsspaltes 1 werden die feinen Partikeln angesaugt und der Bedüsungszone 22 zugeführt, wo diese mit Sprühzusammensetzung benetzt werden und am Wachstumsprozess teilnehmen.

Optional eingebaute Leitbleche 16 stabilisieren die Partikelzirkulation.

Zur kontinuierlichen Prozessführung kann der Apparat optional mit unterschiedlichen Eintragsystemen 13 für Feststoffe ausgerüstet werden. Dadurch können beispielsweise Partikel dem Prozess zugeführt werden, die durch Zerkleinerung von beispielsweise (zu großen) Granulaten gewonnen werden können oder/und aus zu kleinen Granulaten bestehen. Diese Teilchen dienen dann als Granulationskeime oder als Startfüllung zur Verkürzung der Inbetriebnahmezeit. Außerdem können hier Additive in fester Form in den Prozess eingeschleust werden, die in die Granulate eingebettet werden sollen.

Weiterhin kann der Apparat mit Austragsorganen 4 versehen werden, um Partikel aus dem Prozessraum 8 entnehmen zu können. Dies kann beispielsweise durch einen Überlauf oder durch ein volumetrisches Austragsorgan (z.B. eine Zellenradschleuse) oder auch durch einen Schwerkraftsichter (z.B. einen mit Sichtgas beaufschlagten Zick-Zack-Sichter oder einen Steigrohrsichter) erfolgen.

Optional können mechanische Aggregate 27 im Prozessraum 8, jedoch vorzugsweise im Bereich der Rücklaufzone 24 an den geneigten Wänden angebracht werden, um durch Zerkleinerung ausreichend Feinmaterial als Keime für den Granulatbildungsprozess zu erzeugen. Weiterhin kann die Rücklaufzone 24 optional zur Positionierung von Beheizungen oder anderen Wärmeübertragungseinrichtungen 28 genutzt werden. Beispielsweise kann die Apparatewand doppelwandig ausgeführt sein, um diese beispielsweise unter Nutzung von flüssigen oder gasförmigen Wärmeträgem zur Beheizung oder Kühlung der Wände zu verwenden. Somit lassen sich optimale Oberflächentemperaturen einstellen.

Im Prozessraum 8 oder in den darüber liegenden Apparateteilen, der Expansionszone 14 und dem Abluftteil 19, können optional Sprühdüsen 6 angeordnet werden, die vorzugsweise nach unten aber auch teilweise nach oben sprühen. Hier kann ebenfalls die flüssige Formulierung eingedüst werden, um beispielsweise durch Sprühtrocknung / Sprüherstarrung im Apparat Granulationskeime zu erzeugen. Alternativ können über einige der Sprüheinrichtungen 6 und 7 Additive oder andere Komponenten in flüssiger Form eingesprüht und somit in die Granulatstruktur homogen eingebettet werden. Wenn die Sprühdüsen 7 die temperaturbeaufschlagte Zuluftkammer 17 passieren, können optional die flüssigkeitsführenden Teile mit Isolationen oder unterschiedlichen Kühl- oder Heizsystemen 12 versehen werden, um Schädigungen an der flüssigen Formulierung zu unterbinden.

Als Vorteil des erfindungsgemäßen Prozesses ist der sehr einfache Aufbau zu nennen, der eine hohe Betriebssicherheit und Störungsunempfindlichkeit mit sehr guter Reinigungsmöglichkeit verbindet. Somit werden verbesserte Produktionsbedingungen insbesondere hinsichtlich der Pharma- und Hygieneanforderungen bei Produktwechsel geschaffen.

Von Vorteil ist weiterhin, dass Mahlen des eingesetzten Wirkstoffs vor Weiterverarbeitung nicht erforderlich ist. Nach Zumischen von Tablettierhilfsstoffen ist eine Weiterverarbeitung zu Tabletten möglich.

Das erfindungsgemäße Verfahren erlaubt die Herstellung von Granulaten in hoher Ausbeute. Es treten praktische keine Verluste an Wirkstoff auf, da feinteiliges Material in den Prozess zurückgeführt werden kann bzw. bei interner Klassierung erst gar nicht ausgetragen wird.

Gegenstand der vorliegenden Erfindung sind auch die erfindungsgemäß hergestellten Granulate. Die Granulate werden nach dem erfindungsgemäßen Verfahren erhalten und weisen einen d₅₀ von 100 bis 600 µm, vorzugsweise von 150 bis 500 µm, auf.

Zusätzlich dazu oder unabhängig davon weisen die Granulate eine Schüttdichte von 0,400 bis 0,900 g/ml, vorzugsweise von 0,500 bis 0,600 g/ml auf.

Das erfindungsgemäße Produkt ist fließfähig.

Das erfindungsgemäße Produkt weist eine hohe Stabilität auf. Insbesondere kommt es bei der Lagerung nicht zu einer Aggregation oder Verklumpung.

Granulate, wie sie vorstehend erhalten wurden, können auch zu Darreichungsformen mit kontrollierter Freisetzung weiterverarbeitet werden. Solche Darreichungsformen schließen insbesondere Darreichungsformen ein, die den Wirkstoff erst in tieferen Darmabschnitten, wie dem Ileum oder dem Colon, freisetzen. Gemäß einer Ausführungsform werden Granulatteilchen mit einem oder mehreren funktionellen Überzügen zur Steuerung der Wirkstofffreisetzung versehen.

Geeignet gewählte Überzüge lösen sich erst im distalen Teil des Dünndarms (Ileum) und im anschließenden Colon. Sie steuern die Wirkstofffreisetzung so, dass mindestens 60%, vorzugsweise mindestens 70% und insbesondere mindestens 80% des Wirkstoffs im Ileum und im Colon freigesetzt werden.

Solche Überzüge können beispielsweise mit Hilfe eines Wurster-Verfahrens aufgebracht werden.

Als Überzugsmaterialien eignen sich Polymerzusammensetzungen, insbesondere Polymerzusammensetzungen, die zu magensaftresistenten Überzügen führen, die sich bei pH-Werten oberhalb 6,5, wie oberhalb 6,8 und insbesondere oberhalb 7,0 lösen.

Geeignet sind durch Polymerisation von Acrylsäure, Methacrylsäure und deren Estern gewonnene Polymere.

Bevorzugte Polymere sind Methacrylsäure-Methylmethacrylat-Copolymer (1:2), im Handel erhältlich als Eudragit^{®} S 100 (Pulver) und als Eudragit^{®} S 12,5 (organische Lösung), sowie Poly(methylacrylat-co-methylmethacrylat-co-methacrylsäure 7 : 3 : 1, im Handel erhältlich als Eudragit^{®} FS 30 D (wässrige Dispersion).

Diese Polymere können allein oder in Kombination mit anderen Polymeren, wie anderen Eudragit^{®}-Typen, eingesetzt werden, um das gewünschte Freisetzungsverhalten einzustellen.

Den Polymeren können übliche Hilfsstoffe und Zusätze beigemischt werden.

Alternativ zum Überziehen von Granulatteilchen können Darreichungsformen mit kontrollierter Freisetzung auch bereitgestellt werden, indem Granulatteilchen, die nach dem erfindungsgemäßen Verfahren erhalten werden, zu Tabletten verarbeitet oder in Kapseln gefüllt werden, die dann mit einem oder mehreren Überzügen versehen werden. Für die Überzüge gelten die vorstehenden Angaben.

Aus den überzogenen Tabletten oder Kapseln erfolgt die Freisetzung so, dass mindestens 60%, vorzugsweise mindestens 70% und insbesondere mindestens 80% des Wirkstoffs im Ileum und im Colon freigesetzt werden.

### Untersuchungsmethoden

Die Partikelanalysen werden mit dem optischen Bildauswerte-System Camsizer XT (Retsch) durchgeführt. Der CAMSIZER XT nutzt das Prinzip der digitalen Bildverarbeitung. Der dispergierte Partikelstrom passiert zwei LED Stroboskoplichtquellen. Die Schattenprojektionen der Partikel werden von zwei Digitalkameras erfasst. Der Teilchendurchmesser wird als die kürzeste Sehne des gemessenen Satzes maximaler Sehnen einer Teilchenprojektion bestimmt.

Eine Partikelpopulation kann durch einen kumulativen Wert Q3(x) gekennzeichnet werden, der den prozentualen Volumenanteil an Teilchen kleiner als x relativ zum Gesamtvolumen der Teilchen angibt. Der Wert d₅₀ bezeichnet den Wert x, bei dem Q3(x) 50 % ist.

Die Feuchtegehalte des Produkts werden mit dem Feuchteanalysator Sartorius MA 100 (Halogenstrahler; 105 °C und automatischer Abschaltung) bestimmt. Der Feuchtegehalt der erfindungsgemäßen Granulate beträgt typischerweise weniger als 1 Gew.-%.

Zur optischen Beurteilung der Proben werden Aufnahmen mit dem AXIO Mikroskop (Zeiss) angefertigt.

Zur Materialcharakterisierung werden die Versuchsmuster mit dem Röntgendiffraktometer D2 Phaser (Brucker) vermessen.

Schüttvolumen / Schüttdichte werden in einem Standzylinder gemessen. Die Probe wird in den Messzylinder vorsichtig eingefüllt. Sie darf nicht verdichtet (geklopft oder gestoßen) werden.

Nach der Bestimmung von Schüttvolumen / Schüttdichte im Messzylinder wird dieselbe Probe im Zylinder mechanisch gestampft (Stampfvolumeter ERWEKA SVM 20) und das Volumen erneut abgelesen. Dies wird so lange fortgesetzt, bis praktisch keine weiteren Volumenänderungen mehr beobachtet werden.

Schütt- und Stampfdichte werden aus den Messwerten von Masse und Schütt- bzw. Stampfvolumen berechnet.

Der Schüttwinkel ist der Fließneigungswinkel, der sich ergibt, wenn ein frei aus einem Trichter fließendes Produkt auf einer Unterlage einen Kegel bildet. Die Bestimmung erfolgt mit einem Rieseltestgerät RTG01.

### Beispiele

Die Erfindung wird anhand konkreter Anwendungsbeispiele veranschaulicht, ohne dadurch in irgendeiner Weise eingeschränkt zu werden.

### Beispiel 1 - Herstellung von Sprühzusammensetzungen

Das zu verarbeitende Metformin-Hydrochlorid war vollständig zu einem großen Klumpen verklumpt. Der große Wirkstoffklumpen musste zunächst in kleine Stücke gebrochen werden, welche mit Hilfe einer Rotor-Stator-Mühle weiter zerkleinert wurden.

Daraus wurde eine Suspension mit einem Trockenstoffgehalt von 50 % in destilliertem Wasser hergestellt. Die Suspension wurde mit einem Flügelrührer gerührt und anschließend durch ein 500 µm Sieb gegeben, um Düsenblockierungen zu verhindern. Dabei wurde festgestellt, dass immer noch gröbere Bestandteile in der Suspension vorhanden waren. Die Suspension wurde mittels Ultra Turrax T-50 (IKA) für 10 Minuten bei 10.000 U/min erneut aufgerührt. Alle Suspensionen der nachfolgenden Versuche wurden genauso vorbereitet.

In einem weiteren Versuch wurde eine Lösung von Metformin-Hydrochlorid hergestellt. Es war möglich, eine Lösung in Wasser mit einem Trockenstoffgehalt von 28 % zu erhalten. Auch hierfür musste der Ultra Turrax verwendet werden.

### Beispiel 2 - Herstellung eines Metformin-Hydrochlorid-Produkts ohne Zusatz von Hilfsstoffen

Die Granulationsversuche wurden kontinuierlich in einer Laboranlage mit Strahlschichteinsatz durchgeführt.

Mit einer Bottomspraydüse (Zweistoffdüse; Düsenlufttemperatur nicht beheizt) wurde die Sprühzusammensetzung verdüst.

Es wurde eine Metformin-Hydrochlorid-Lösung in Wasser mit einem Trockenstoffgehalt von 28 % in den Apparat eingesprüht.

Die Sprühzusammensetzung wurde vom Vorlagebehälter (5 l Behälter; nicht beheizt) mit einer Schlauchpumpe zu der Düse befördert.

Oberhalb der Strahlschicht waren Filter angeordnet. Sie wurden regelmäßig durch Druckluftstöße abgereinigt, sodass der Staub im Prozessraum blieb.

Die Prozessluft wurde durch den drehzahlgeregelten Abluftventilator gefördert. Für das Aufheizen der Luft wurde ein elektrisches Heizregister genutzt.

Der Produktaustrag wurde mittels Luftstrom im Zick-Zack-Sichter geregelt, sodass bei stabilen Betriebsbedingungen die gleiche Menge Trockenstoff als staubfreies Granulat ausgetragen wurde, die mit der Sprühzusammensetzung zugeführt wurde.

Der Feinstaub aus dem Sichter wurde zurück in die Prozesskammer gefördert.

Nach dem beschriebenen Verfahren konnten Wirkstoffpellets bestehend aus 100% Wirkstoff aus der Lösung erhalten werden.

Der Prozess lief sehr stabil.

Zunächst wurden kleine Partikel erzeugt (Probe A; d₅₀ = 123,2 µm).

Anschließend wurden der Sprühdruck gesenkt und die Sprührate erhöht, um das Partikelwachstum zu fördern. Es konnten dann größere Partikel erzeugt werden (Probe B; d₅₀ = 300,9 µm).

### Beispiel 3 - PVP und Partikelgröße

Für dieses Beispiel wurde eine Suspension von Metformin-Hydrochlorid in Wasser mit 5 Gew.-% PVP Kollidon K-30 (bezogen auf den Trockenstoff) hergestellt. Die Suspension hatte einen Trockenstoffgehalt von 51,3 %. Der Prozess wurde mit der Restschicht des vorherigen Versuchs gestartet.

Es wurde eine Düse von 2,0 mm verwendet. Während des Sprühens wurde die Suspension gerührt.

Das dem Prozessraum ausgetragene Produkt wies einen d₅₀ von 197,6 µm auf.

Anschließend wurden der Sprühdruck gesenkt und die Sprührate erhöht, um das Partikelwachstum zu fördern. Das nach Austausch der Schichtmasse ausgetragene Produkt wies einen d₅₀ von 423,6 µm auf.

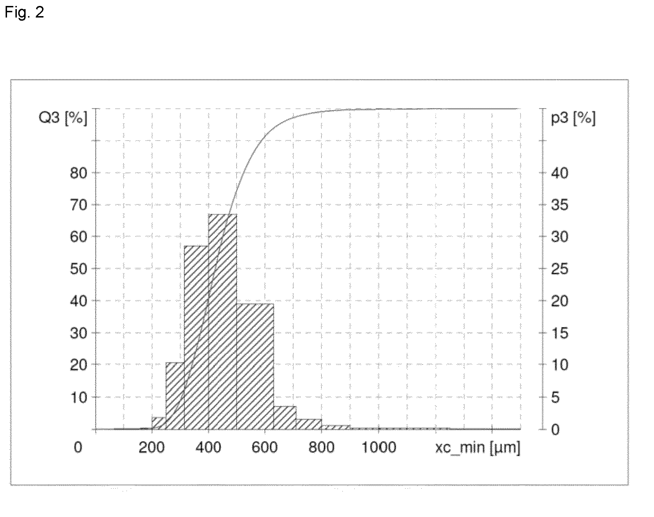

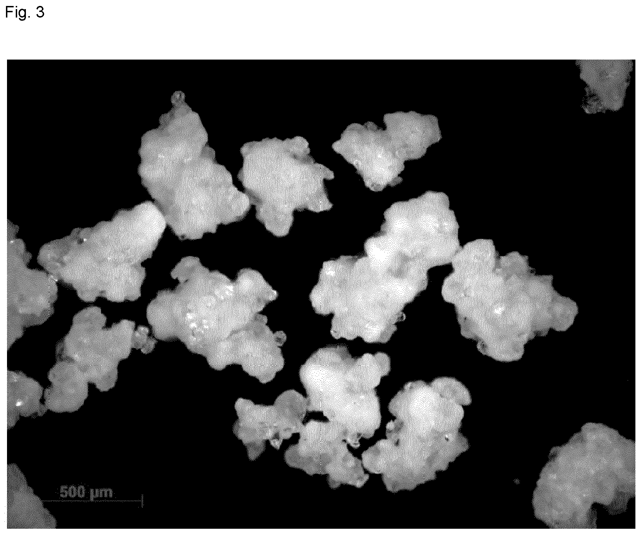

Die Teilchengrößenverteilung des Produkts ist in Fig. 2 gezeigt. Die mikroskopische Aufnahme einer Probe ist in Fig. 3 gezeigt. Die erhaltenen Produktpartikel stellen Aggregate aus fest miteinander verbundenen Kügelchen dar.

Es war möglich, unterschiedliche Partikelgrößen zu produzieren mit einem PVP Gehalt von 5% im Endprodukt.

### Beispiel 4 - Durchsatz

In diesem Test sollte der Prozess weiter optimiert werden. Aus diesem Grund wurde versucht, die Suspension von Metformin-Hydrochlorid und PVP in Wasser höher zu konzentrieren. Es wurde eine Suspension mit einem Trockenstoffgehalt von 69,4 % erhalten. Bezogen auf den Trockenstoffgehalt waren wieder 5 % PVP in der Suspension.

Trotz der hohen Viskosität war es möglich, die Suspension zu versprühen. Aufgrund der geringeren Menge an Wasser, das verdampft werden musste, konnte eine große Durchsatzsteigerung erzielt werden (1,3 kg/h in Beispiel 3; 2,9 kg/h im vorliegenden Beispiel).

Zunächst wurde wieder eine kleine Partikelgröße produziert (d₅₀ = 186,8 µm). Anschließend wurden größere Partikel hergestellt (d₅₀ = 475,3 µm). Der Prozess konnte ohne Düsenblockierungen oder andere Probleme beendet werden.

### Beispiel 5 - Variation des Bindemittelgehalts

Der PVP Gehalt wurde für diesen Versuch von 5 % auf 2,5 % verringert (bezogen auf den Trockenstoff). Die Konzentration der Suspension wurde beibehalten (68,8 %). Nach Austausch der Schichtmasse wurden zunächst kleine Teilchen (d₅₀ = 184,7 µm) und anschließend gröbere Teilchen (d₅₀ = 269,2 µm) hergestellt.

Für einen weiteren Versuch wurde wieder eine Suspension hergestellt (Trockenstoffgehalt 70,2 %). Diesmal wurden nur 1 % PVP (bezogen auf den Trockenstoff) hinzugegeben. Auch hier wurde wieder zunächst eine kleine Partikelgröße produziert (d₅₀ = 165,3 µm). Später wurden größere Partikel (d₅₀ = 230,5 µm) produziert.

Es können also wiederum Teilchen verschiedener Größe hergestellt werden.

### Beispiel 6 - Fließfähigkeit

Es wurden verschiedene Parameter bestimmt, die Rückschlüsse auf die Fließfähigkeit von Produkten erlauben.

Der reine Wirkstoff wurde für die Messung leicht desagglomeriert, damit die Untersuchung überhaupt stattfinden konnte.

Der Hausner-Faktor wurde ermittelt als das Verhältnis von Stampfdichte zu Schüttdichte. Bei Werten nahe 1 ist eine gute Dosiergenauigkeit zu erwarten, bei Werten deutlich über 1 kann die Dosiergenauigkeit von Erschütterungen abhängen. Im vorliegenden Fall zeigt eine Verringerung des Hausner-Faktors für die erfindungsgemäßen Proben im Vergleich mit dem Rohstoff eine Verbesserung der Dosiergenauigkeit.

Der Carr-Index wurde nach der Formel 100 x (Schüttvolumen - Stampfvolumen)/Schüttvolumen ermittelt. Kleinere Carr-Indizes zeigen ein besseres Fließverhalten. Ein Wert kleiner 15 zeigt ein frei fließendes Produkt.

Ein kleiner Schüttwinkel zeigt ein gutes Fließverhalten.

**Tabelle 1**

| **Probe** | **Schüttvolume n** | **Einwaage** | **Schütt -dichte** | **Stampfvolumen** | **Stampfdichte** | **Hausner Faktor** | **Carr Inde x** | **Schütt - winkel** |
|---|---|---|---|---|---|---|---|---|
| | ml | g | g/ml | ml | g/ml | | | ∘ |
| 95 % Metf. / 5 % PVP | 174 | 98,9 | 0,568 | 163 | 0,607 | 1,067 | 6,322 | 6,22 |
| 97,5 % Metf. / 2,5 % PVP | 168 | 100 | 0,595 | 156 | 0,641 | 1,077 | 7,143 | 6,11 |

### Beispiel 7 - Stabilität

Erfindungsgemäße Proben wurden für 3 Monate bei Raumtemperatur in versiegelten Kunststoffbeuteln gelagert. Die gute Fließfähigkeit blieb erhalten.

### Beispiel 8 - Herstellung von Tabletten

Es wurden Tabletten unter Verwendung von Metformin-Hydrochlorid-Produkten, wie sie in einigen der vorstehenden Beispiele erhalten wurden, hergestellt.

Zusammensetzung und Eigenschaften der eingesetzten Metformin-Hydrochlorid-Produkte sind in der nachstehenden Tabelle 2 angegeben:

**Tabelle 2**

| Produkt | Gehalt PVP | d₅₀ | Schüttdichte | Restfeuchte |
|---|---|---|---|---|
| P1 | 5 % | 197,6 µm | n.b. | 0,71 % |
| P2 | 5 % | 423,6 µm | 0,510 g/ml | 0,69 % |
| P3 | 2,5 % | 184,7 µm | 0,582 g/ml | 0,29 % |
| P4 | 2,5 % | 269,2 µm | 0,529 g/ml | 0,38 % |
| P5 | - | 123,2 µm | 0,851 g/ml | 0,23 % |
| P6 | - | 300,9 µm | n.b. | 1,06 % |

Um Tablettiermischungen herzustellen, wurden jeweils 1000 Gewichtsteile Metformin-Hydrochlorid-Produkt mit 3 Gewichtsteilen Magnesiumstearat als Trennmittel und 31 Gewichtsteilen Croscarmellose-Natrium (AcDiSol^{®}) als Zerfallsmittel gemischt.

Zur Herstellung von biconvexen Tabletten wurde eine Tablettenpresse von Fette (102i) mit einem Stempeldurchmesser von ca. 10 mm verwendet. Die Fülltiefe betrug 9 mm. Es wurde mit einer Geschwindigkeit von 10000 Tabletten pro Stunde gearbeitet.

Weitere Parameter sind in der nachstehenden Tabelle 3 angegeben.

**Tabelle 3**

| Nr. | Produkt | Steghöhe mm | Vorpresskraft kN | Steghöhe mm | Hauptpresskraft kN | Masse mg |
|---|---|---|---|---|---|---|
| T1.1 | P1 | 4,7 | 3 | 4,2 | 6,8 | 382 |
| T1.2 | P1 | 5,2 | 1,4 | 4,7 | 3 | 381 |
| T1.3 | P1 | 4,3 | 4 | 3,8 | 9,4 | 372 |
| T2.1 | P2 | 4,7 | 1,4 | 4,2 | 3 | 346 |
| T2.2 | P2 | 4 | 2,5 | 3,5 | 6,7 | 328 |
| T2.3 | P2 | 3,5 | 4,6 | 3,2 | 9,3 | 320 |
| T3.1 | P3 | 4,2 | 4 | 3,75 | 9,1 | 370 |
| T3.2 | P3 | 4,3 | 3 | 3,85 | 7 | 376 |
| T3.3 | P3 | 4,7 | 1,6 | 4,3 | 3,1 | 359 |
| T4.1 | P4 | 4,9 | 1,5 | 4,5 | 3 | 373 |
| T4.2 | P4 | 4 | 4 | 3,5 | 9 | 350 |
| T4.3 | P4 | 3,9 | 2,7 | 3,5 | 6,2 | 332 |
| T5.1 | P5 | 5,5 | 3,3 | 5 | 7,2 | 453 |
| T5.2 | P5 | 5,2 | 4,4 | 4,7 | 10,5 | 445 |
| T6.1 | P6 | 4,9 | 3,8 | 4,4 | 11 | 442 |
| T6.2 | P6 | 5 | 1,3 | 4,5 | 4,7 | 414 |

In allen Fällen konnten Tabletten erhalten werden, die annehmbare Zerfallszeiten aufwiesen.

### Beispiel 9 - Herstellung von überzogenen Granulatteilchen

Überzogene Granulatteilchen werden aus einem Metformin-Hydrochlorid-Granulat hergestellt. Der Überzug soll sicherstellen, dass die Wirkstofffreisetzung überwiegend im Ileum und im Colon erfolgt.

Formulierung einer beispielhaften Überzugssuspension:

| | |
|---|---|
| EUDRAGIT^{®} FS 30 D (erhältlich von Evonik Roehm GmbH, Darmstadt, DE) | 2000 g |
| Talc (erhältlich von Merck KGaA, Darmstadt, DE) | 300 g |
| Triethylcitrat (TEC) (erhältlich von Vertellus Inc., Greensboro, USA) | 37,5 g |
| Wasser (entmineralisiert) | 2350 g |

Zur Herstellung der Überzugssuspension werden EUDRAGIT^{®} FS 30 D, Talc und TEC unter Verwendung eines Flügelrührers (IKA GmbH & Co.KG, Staufen, DE) gemischt. Die Suspension wird durch ein 0,1 mm Sieb passiert.

Die Suspension weist einen Feststoffgehalt von 20,0 % und einen Polymergehalt von 12,8 % auf.

Die Suspension wird durch ein Wirbelschichtverfahren auf ein Metformin-Hydrochlorid-Granulat (d₅₀ = 350 µm) aufgebracht. Dazu wird eine Wirbelschichtanlage Glatt GPCG 1 (Glatt GmbH, Binzen, DE) mit einer 1,2 mm-Düse (Top-Spray) und einem Atomisierungsluftdruck von 2 bar verwendet. Weitere Prozessparameter sind eine Sprührate von 7-10 g/min/kg, eine Einlasslufttemperatur von 38-40 °C und eine Auslasstemperatur von 26-30 °C.

Die Suspension wird so lange eingesprüht, bis 30,0 Gew.-% Polymer, bezogen auf das eingesetzte Metformin-Hydrochlorid-Granulat, aufgebracht sind.

Anschließend wird das Endprodukt in der Anlage getrocknet. Um eine Agglomeration zu verhindern, erfolgt ein Zusatz von 0,5 % Aerosil^{®} 200 (pyrogenes Siliciumdioxid) vor dem Trocknen.

### Bezugszeichen

- 1: Spaltöffnung(en)
- 2: Gasstrahl(en)
- 3: Umlenkteil
- 4: Austragsorgan
- 5: Seitenwand
- 6: Sprühdüse(n) in beliebige Richtungen sprühend
- 7: Sprühdüse(n) aufwärts sprühend
- 8: Prozessraum
- 9: Querschnitt einer Prozessstufe
- 10: Prozessgas
- 11: Abgas
- 12: Isolation mit Kühl- oder Heizsystem
- 13: Eintragssystem
- 14: Expansionszone
- 15: Feststoffzirkulation
- 16: Leitblech(e)
- 17: Zuluftkammer
- 18: Druckluftimpulse
- 19: Abluftteil
- 20: feststoffbeladene Abluft
- 21: abgetrennter und zurückgeführter Feststoff
- 22: Bedüsungszone
- 23: Partikelaustrittes aus dem Gasstrahl
- 24: Rücklaufzone
- 25: Entstaubungsanlage
- 26: Zuführungen
- 27: Mechanische Aggregate zur Zerkleinerung
- 28: Wärmeübertragungseinrichtungen
- 29: Seitenwand

## Patentansprüche

1. Verfahren zur kontinuierlichen Herstellung eines Wirkstoffgranulats, das folgende Stufen umfasst:
(a) Herstellen einer Sprühzusammensetzung, indem ein Wirkstoff und ggf. ein oder mehrere Hilfsstoffe in einer Flüssigkeit gelöst oder dispergiert werden;
(b) Bereitstellen von festen Teilchen in einem Prozessraum;
(c) Einführen von Tröpfchen aus der Sprühzusammensetzung in eine Eindüsungszone des Prozessraums, in dem die Flüssigkeit verdampft;
(d) wiederholtes Vorbeiführen der festen Teilchen an eingesprühten Tröpfchen in dem Prozessraum mit Hilfe eines Prozessgasstrahls, so dass zumindest ein Anteil der Tröpfchen, die bereits einen Teil der enthaltenen Flüssigkeit verloren haben können, mit festen Teilchen in Kontakt kommt und durch Anlagerung größere feste Teilchen gebildet werden;
(e) Entnehmen des Wirkstoffgranulats aus dem Prozessraum in Form von festen Teilchen,
wobei der Wirkstoff Metformin oder ein Säureadditionssalz von Metformin, insbesondere Metformin-Hydrochlorid, umfasst.

2. Verfahren nach Anspruch 1, wobei es sich bei dem Wirkstoff um Metformin-Hydrochlorid handelt.

3. Verfahren nach Anspruch 1 oder 2, wobei es sich bei der Flüssigkeit um Wasser handelt.

4. Verfahren nach einem der vorstehenden Ansprüche, wobei die Sprühzusammensetzung zusätzlich ein Bindemittel enthält.

5. Verfahren nach einem der vorstehenden Ansprüche, wobei der Trockenstoffgehalt der Sprühzusammensetzung mindestens 25 Gew.-% beträgt.

6. Verfahren nach Anspruch 5, wobei der Anteil des Wirkstoffs an dem Trockenstoffgehalt mindestens 70 Gew.-% beträgt.

7. Verfahren nach einem der vorstehenden Ansprüche, wobei es sich bei der Sprühzusammensetzung um eine Suspension handelt.

8. Verfahren nach einem der vorstehenden Ansprüche, wobei die Bereitstellung von Teilchen gemäß Stufe (b) durch Einsprühen der Sprühzusammensetzung in den leeren Prozessraum und Verdampfen der Flüssigkeit in dem Prozessraum erfolgt.

9. Verfahren nach einem der vorstehenden Ansprüche, wobei in Stufe (e) Teilchen entnommen werden, die eine festgelegte Größe erreicht haben.

10. Verfahren nach einem der Ansprüche 1 bis 8, wobei feste Teilchen, die eine festgelegte Größe nicht erreicht haben, in den Prozessraum zurückgeführt werden.

11. Verfahren nach einem der vorstehenden Ansprüche, wobei zu große Teilchen nach Zerkleinern in den Prozessraum zurückgeführt werden.

12. Verfahren nach einem der vorstehenden Ansprüche, bei dem die Teilchengröße der entnommenen Teilchen, ausgedrückt als d₅₀-Wert, 100 bis 600 µm beträgt.

13. Wirkstoffgranulat, hergestellt nach einem der vorstehenden Ansprüche, wobei die Teilchengröße, ausgedrückt als d₅₀-Wert, 100 bis 600 µm beträgt.

14. Wirkstoffgranulat, hergestellt nach einem der Ansprüche 1 bis 12, das mit einem Überzug zur kontrollierten Freisetzung versehen ist.

15. Wirkstoffgranulat nach Anspruch 14, wobei der Überzug die Freisetzung so steuert, dass mindestens 60%, vorzugsweise mindestens 70% und insbesondere mindestens 80% des Wirkstoffs im Ileum und im Colon freigesetzt werden.

16. Verwendung eines Wirkstoffgranulats, hergestellt nach einem der Ansprüche 1 bis 12, zur Herstellung einer Tablette oder Kapsel.

17. Verwendung nach Anspruch 16, wobei die Tablette oder Kapsel mit einem Überzug zur kontrollierten Freisetzung versehen wird.

18. Verwendung nach Anspruch 17, wobei die Tablette oder Kapsel mindestens 60%, vorzugsweise mindestens 70% und insbesondere mindestens 80% des Wirkstoffs im Ileum und im Colon freisetzt.

## Claims

1. A process for the continuous production of an active ingredient granulate, comprising the following steps:
(a) preparing a spray composition by dissolving or dispersing an active ingredient and optionally one or more excipients in a liquid;
(b) providing solid particles in a process space;
(c) introducing droplets of the spray composition into an injection zone of the process space in which the liquid evaporates;
(d) repeated guiding of the solid particles past the sprayed droplets in the process space with the aid of a process gas jet, so that at least a portion of the droplets, which may have already lost part of the liquid contained, comes into contact with solid particles and larger solid particles are formed through agglomeration;
(e) removing the active ingredient granulate from the process space in the form of solid particles,
wherein the active ingredient comprises metformin or an acid addition salt of metformin, in particular metformin hydrochloride.

2. The process according to claim 1, wherein the active ingredient is metformin hydrochloride.

3. The process according to claim 1 or 2, wherein the liquid is water.

4. The process according to any one of the preceding claims, wherein the spray composition additionally contains a binder.

5. The process according to any one of the preceding claims, wherein the dry matter content of the spray composition is at least 25% by weight.

6. The process according to claim 5, wherein the proportion of the active ingredient in the dry matter content is at least 70% by weight.

7. The process according to any one of the preceding claims, wherein the spray composition is a suspension.

8. The process according to any one of the preceding claims, wherein the provision of particles according to step (b) takes place by spraying the spray composition into the empty process space and evaporating the liquid in the process space.

9. The process according to any one of the preceding claims, wherein in step (e) particles which have reached a predetermined size are removed.

10. The process according to any one of claims 1 to 8, wherein solid particles which have not reached a specified size are returned to the process space.

11. The process according to any one of the preceding claims, wherein excessively large particles are returned to the process space after comminution.

12. The process according to any one of the preceding claims, in which the particle size of the particles removed, expressed as the d₅₀ value, is 100 to 600 µm.

13. An active ingredient granulate, produced according to any one of the preceding claims, wherein the particle size, expressed as d₅₀ value, being 100 to 600 µm.

14. The active ingredient granulate, produced according to any one of claims 1 to 12, provided with a coating for controlled release.

15. The active ingredient granulate according to claim 14, wherein the coating controls the release so that at least 60%, preferably at least 70%, and in particular at least 80% of the active ingredient are released in the ileum and in the colon.

16. A use of an active ingredient granulate, produced according to one of claims 1 to 12, for producing a tablet or capsule.

17. The use according to claim 16, wherein the tablet or capsule is coated with a controlled release coating.

18. The use according to claim 17, wherein the tablet or capsule releases at least 60%, preferably at least 70% and in particular at least 80% of the active ingredient in the ileum and in the colon.

## Revendications

1. Procédé de fabrication en continu d'un granulé de substance active, comprenant les étapes suivantes :
(a) préparation d'une composition à pulvériser en dissolvant ou en dispersant une substance active et, le cas échéant, un ou plusieurs adjuvants dans un liquide ;
(b) mise à disposition de particules solides dans une chambre de traitement ;
(c) introduction de gouttelettes provenant de la composition à pulvériser dans une zone d'injection de la chambre de traitement dans laquelle le liquide s'évapore ;
(d) passage répété des particules solides devant les gouttelettes pulvérisées dans la chambre de traitement à l'aide d'un jet de gaz de traitement, de telle sorte qu'au moins une partie des gouttelettes qui ont déjà pu perdre une partie du liquide qu'elles contiennent entre en contact avec des particules solides et que des particules solides plus grosses se forment par agglomération ;
(e) prélèvement des granulés de substance active de la chambre de traitement sous forme de particules solides,
la substance active comprenant de la metformine ou un sel d'addition d'acide de la metformine, en particulier le chlorhydrate de metformine.

2. Procédé selon la revendication 1, dans lequel le principe actif est le chlorhydrate de metformine.

3. Procédé selon la revendication 1 ou 2, dans lequel le liquide est de l'eau.

4. Procédé selon l'une des revendications précédentes, dans lequel la composition à pulvériser contient en outre un liant.

5. Procédé selon l'une des revendications précédentes, dans lequel la teneur en matière sèche de la composition à pulvériser est d'au moins 25 % en poids.

6. Procédé selon la revendication 5, dans lequel la proportion de substance active dans la teneur en matière sèche est d'au moins 70 % en poids.

7. Procédé selon l'une des revendications précédentes, dans lequel la composition à pulvériser est une suspension.

8. Procédé selon l'une des revendications précédentes, dans lequel la mise à disposition de particules selon l'étape (b) s'effectue par pulvérisation de la composition à pulvériser dans l'espace de traitement vide et évaporation du liquide dans l'espace de traitement.

9. Procédé selon l'une des revendications précédentes, dans lequel, à l'étape (e), on prélève les particules qui ont atteint une taille déterminée.

10. Procédé selon l'une des revendications 1 à 8, dans lequel les particules solides qui n'ont pas atteint une taille définie sont renvoyées dans la chambre de traitement.

11. Procédé selon l'une des revendications précédentes, dans lequel les particules trop grosses sont renvoyées dans la chambre de traitement après avoir été broyées.

12. Procédé selon l'une des revendications précédentes, dans lequel la taille des particules prélevées, exprimée en valeur d 50, est comprise entre 100 et 600 µm.

13. Granulés de substance active, fabriqués selon l'une des revendications précédentes, la taille des particules, exprimée en valeur d₅₀, étant comprise entre 100 et 600 µm.

14. Granulés de substance active, fabriqués selon l'une des revendications 1 à 12, qui sont pourvus d'un enrobage pour une libération contrôlée.

15. Granulés de substance active selon la revendication 14, dans lesquels le revêtement contrôle la libération de telle sorte qu'au moins 60 %, de préférence au moins 70 % et en particulier au moins 80 % de la substance active soient libérés dans l'iléon et dans le côlon.

16. Utilisation d'un granulé de principe actif, fabriqué selon l'une des revendications 1 à 12, pour la fabrication d'un comprimé ou d'une capsule.

17. Utilisation selon la revendication 16, dans laquelle le comprimé ou la capsule est pourvu d'un enrobage à libération contrôlée.

18. Utilisation selon la revendication 17, dans laquelle le comprimé ou la capsule libère au moins 60 %, de préférence au moins 70 % et en particulier au moins 80 % du principe actif dans l'iléon et dans le côlon.
